# EUROPEAN PATENT APPLICATION

(11) **EP 3 892 314 A1**
(43) Date of publication of application: **13.10.2021**
(21) Application number: 20168226.7
(22) Date of filing: 06.04.2020
(51) Int. Cl.: A61M 1/36, A61M 25/00

(54) **INFLOW CANNULA**

(71) Applicant: BIOTRONIK SE & Co. KG, 12359 Berlin (DE)
(72) Inventor: NOLLERT, Georg, 82064 Strasslach (DE); KOERNER, Sebastian, 10713 Berlin (DE); LANG, Volker, 12161 Berlin (DE)
(74) Representative: Biotronik Corporate Services SE

(57) **Abstract**

The present invention is generally directed to an inflow cannula (1) having a tubular body (4) comprising at least one lateral hole (3) positioned at a predefined section (6) of the tubular body (4), wherein the inflow cannula (1) further comprises at least one cage (20) and a sheath (10) concentrically arranged around the tubular body (4), wherein the sheath (10) is movable between a first position and a second position, wherein the at least one cage has a contracted state and an expanded state. To achieve a minimum diameter of inflow cannula (1) together with a high stiffness, the at least one cage (20) takes a conical form and is positioned in the predefined section (6) of the tubular body (4) in its expanded state, wherein in its contracted state the at least one cage (20) is arranged between the outer surface of the tubular body (4) and the sheath (10) when the sheath (10) is in its first position, and wherein the cage (20) is adapted to expand and takes its expanded state when the sheath (10) is moved from its first position into its second position.

## Description

The present invention is generally directed to an inflow cannula that can be used for extraction of bodily fluids, for example for arterial and venous blood. Such an inflow cannula may comprise a tubular body with at least one lateral inflow through hole or bore (in the following: hole) positioned at a predefined section of the tubular body.

Inflow cannulas are well known and used for example for the extraction of bodily fluids such as blood. Generally, there are several types of cannulas for different medical applications. However, inflow cannulas penetrate the human or animal body during medical application and withdraw the predefined bodily fluid from the human or animal vessel or organ.

An inflow cannula usually comprises a head-on hole, wherein the cannula also with at least one lateral hole is known in the prior art to enhance the inflow volume. The lateral hole is positioned at a predefined section, usually a distal section, of the tubular body of the inflow cannula. An inflow cannula of circulatory support systems (e.g. Extracorporeal Membrane Oxygenation (ECMO), Extracorporeal Lung Assist (ECLA, ELA), Cardiopulmonary Bypass (CPB), Ventricular Assist Devices (VAD)) and also of filtration systems (e.g. dialysis, hemofiltration) tends to attach to the vessel or organ wall or other anatomical structures such as the mitral valve. This is particularly the case when negative pressure (suction) is applied to increase flow. For example, too much vacuum or suction results in the vessel or organ being collapsed down around the cannula's tubular body rendering the holes incapable of providing a passage way for fluid flow. As soon as the cannula attaches to the vessels, organs, ventricle walls or other anatomical structures, the lateral holes of the cannula are at least partially closed, which leads to a decreased flow, to a limited function of the system (e.g. reduced cardiac output in VADs), injured vessel or organ structures and even to serious hemolysis particularly in high flow pumps.

To avoid these problems, it is known to include a metal spacer separating the vessels, walls of organs or other anatomical structures from the cannula. However, if an inflow cannula is to be used in a minimal invasive treatment, known spacers significantly increase the diameter of the cannula thereby causing injury of the vessels and organs particular during insertion and withdrawal of the cannula.

Thus, a venous drainage cannula especially for cardiopulmonary bypass that prevents vena cava collapse, which has an expandable scaffolding at its distal end, is shown in document US 6,673,042 B1. The cannula comprises distal ports for the extraction of bodily fluids and a sheath covering a scaffolding to compress the scaffolding into a collapsed state and provides a smooth outer surface for insertion and withdrawal of the venous catheter, wherein in the scaffolding is deployed by withdrawal of the sheath. The known cannula comprises a scaffolding with a balloon-like form in the deployed state comprising a multiplicity of filaments arranged in a braided configuration which is sized and configured to support an inner surface of a vein when in an expanded condition.

Document US 2016/0158489 A1 discloses a self-expanding cannula with a self-expanding wire frame, wherein the expanded wire frame, which comprises a stent-like structure, provides radial support to prevent a drainage canal from collapsing as fluid is drained from the patient.

Further, in document EP 1 839 601 A1 another self-expanding cannula is disclosed. The cannula is made of braided-wire and expands in a tubular shape once a flexible membrane is retracted.

Therefore, it is an object of the present invention to provide a cannula, especially for minimal invasive access, which uses the known advantages of the arrangement of lateral holes to increase the flow, which can be produced easily and cost-effectively, has a minimum diameter and an improved stiffness.

This object is solved by an inflow cannula with the features of claim 1.

An inflow cannula according to the present invention comprises a tubular body with a distal end and a proximal end. The tubular body may comprise a single lumen or a double lumen thereby realizing a single lumen cannula (SLC) or a double lumen cannula (DLC). The inner cavity or lumen is provided for withdrawal of bodily fluid during treatment. Further, the tubular body has at least one lateral inflow hole positioned at a predefined section of the tubular body, wherein the inflow cannula further comprises at least one cage and a sheath concentrically arranged around the tubular body, wherein the sheath is movable between a first and a second position. The at least one cage has a contracted state and an expanded state, wherein the at least one cage takes a conical form and is positioned in the predefined section of the tubular body in its expanded state. In its contracted state the at least one cage is arranged between the outer surface of the tubular body and the sheath when the sheath is in its first position, and wherein the cage is adapted to expand and takes its expanded state when the sheath is moved from its first position into its second position.

In the following description, the terms "distal" and "proximal" refer to the respective distance from the person that is inserting the cannula into the patient. Further, the tubular body, the cage and the sheath each have a longitudinal axis which is the same or at least parallel to each other. The longitudinal direction is parallel to the longitudinal axis of these elements.

In one embodiment, the through-going lateral inflow holes are positioned closer to the distal end of the tubular body, wherein the cannula is preferably used for arterial or venous medical treatment. Nevertheless, according to the invention the lateral inflow holes may also be accommodated in a centered position with regard to the distal end or the proximal end of the tubular body or more proximal. The lateral holes need not necessarily to be round. Oval or angular lateral holes are also conceivable. Further, the predefined section of the tubular body is defined by the outermost circumference of the most distal and most proximal lateral hole of a predefined group of lateral holes such that one predefined section comprises all lateral holes of this predefined group.

In one embodiment, the tubular body may comprise one head-on hole or a plurality of head-on holes at its distal front end.

The inflow cannula comprises a sheath which comprises or is fully made of a biocompatible material such as biocompatible polymers as, for example, polyvinylchloride, polyurethane or silicone. Moreover, the sheath is movable between a first and a second position in longitudinal direction, wherein the sheath at least partially covers the cage, when the sheath is in its first position. Therefore, the cage is contracted/collapsed, when the sheath is in its first position. In its initial, contracted state the cage may take a tubular form or the form of a hollow cylinder. Once the sheath is retracted, i.e. moved towards its second position, the cage expands and takes a conical form. Thus, the first position of the sheath may be more distal than the second position. If the sheath is not fully retracted, i.e. only partly retracted, it may be possible that only one section, for example a distal section, of the cage expands conically, wherein the remaining section of the cage covered by the sheath remains in its initial position that may be the form of a hollow cylinder. If the sheath is fully retracted, the cage expands to a conical form, which is taken by the largest section of the cage in longitudinal direction. The advantage of the conical form of the cage with its greater diameter at its distal end is, in particular, that the load acting on the cage can be directed towards the cannula and thus to the strongest component of the system. Further, using a conical form of the cage has additional benefits of an improved stiffness, which results in the cage being more stable against external loads, as well as of a simplified manufacturing. Additionally, a conical form or shape of the cage may be understood as a funnel form or shape, wherein the smallest diameter is equal to or greater than the outer diameter of the tubular body of the inflow cannula. Thus, depending on its orientation within the vessel etc., the cage may improve the inflow rate as the bodily fluid is conducted more direct to the lateral holes. Further, the inflow rate may even be increased due to the natural flow direction of the bodily fluid, wherein the fluid is also conducted directly by the cage to the lateral inflow holes. The cross section of the cage may be circular or oval.

In one embodiment, the sheath is a fully solid piece. In another embodiment may comprise a grid or other net-like structure with a plurality of partly or fully through-going recesses.

In one embodiment, the cage of the inflow cannula comprises struts. Those struts may all have the same or different width or thickness. Further, the width or thickness may depend on the diameter or shape of the lateral holes. Furthermore, the cage may be formed from one strut or from a plurality of struts. The number of struts may correspond to the longitudinal length of the predefined section or the number of lateral holes covered by the respective cage.

In one embodiment, a first plurality of struts extends in a longitudinal direction of the tubular body and a second plurality of struts extends in a circumferential direction of the cage. In particular, there may be at least two struts extending in a longitudinal direction of the tubular body as well as at least two struts extending in a circumferential direction of the cage, wherein the circumferential struts enclose an area with different diameters. In one embodiment, the most distal circumferential strut covers the greatest diameter and the most proximal strut covers the smallest diameter. In addition, there may be more struts as already mentioned above. Furthermore, there may be struts oriented in other directions that may even cross the above mentioned struts such as bracings. The struts may be inclined/sloping with regard to the longitudinal or circumferential direction. Such struts may further increase the stiffness of the cage.

Preferably, the cage comprises or is fully made of a shape memory and/or superelastic material, for example Nitinol. The use of other shape memory and/or superelastic materials than Nitinol is possible as long as they are biocompatible, for example a copper zinc alloy. Using a cage comprising a shape memory and/or supereleastic material, allows instantaneous expansion of the cage into a desired shape once the sheath is retracted. Hence, the cage is self-expanding and may be biased. Furthermore, shape memory materials are known for their great work performance, their potential for the integration of functions, their simple and compact design, their low weight, and that there are no moving parts required to change their form. Further advantages of shape memory materials are well known in the prior art.

The tubular body comprises or is fully made of the following biocompatible materials, such as, for example, polyvinylchloride, polyurethane or silicone.

The tubular body, the cage and/or the sheath may be flexible in order to adapt to the form of the surrounding anatomical structures.

In one embodiment the inflow cannula may further comprise a Peltier element attached to and thermally connected to the cage, for example attached to one of its struts. The Peltier element is used to change the temperature of the cage. Thus, the cage comprising a shape memory material may be activated by the increased temperature when entering the human or animal body and thereby may be expanded immediately when the sheath is retracted. If the cage is to be returned to its contracted state at the end of the medical treatment, the Peltier element may lower the temperature of the cage, e.g. to a temperature or close to the temperature, at which the shape memory and/or superelastic exhibits a maximal elastic behavior. Consequently, the cage becomes more elastically and/or flexible, which makes it easier to slip the sheath back over the cage. Other scenarios, wherein the Peltier element is used at any time during the medical application to change the temperature of the cage and thus to either stiffen or to elasticize the cage, are of course covered by this embodiment. To activate the Peltier element, an electrical connector/conductor is provided electrically connecting the Peltier element with a power supply. The Peltier element may also be arranged at the inner surface of the tubular body and thus changing the temperature of the cage in its contracted state, wherein in this state the at least one cage is thermally connected to the Peltier element. Thus, the cage may adapt to the temperature of the tubular body of the inflow cannula. Instead of providing just one Peltier element, there may be a plurality of Peltier elements used and therefore attached to the cage.

In one embodiment, the inflow cannula may further comprise at least one sensor and/or transmitter attached to the cage, for example to one of its struts, or the outer surface of the tubular body. Such transmitter may be used if the information processing is extracorporeally provided. The sensor and/or transmitter may be electrically connected to a power supply. The sensor may detect the state of the cage. Other types of sensors may determine the temperature of the cage, the flow rate into the cannula or any other types of physical or biological information regarding the system and/or the medical treatment. A sensor does not necessarily have to be attached to the cage but alternatively to the tubular body.

In one embodiment, the tubular body of the cannula may comprise at least one contact element, for example at the outer surface of the tubular body, wherein the at least one sensor is adapted to detect an electrical connection of the at least one cage with the at least one contact element. Hence, it might be detected, whether the cage is in its expanded or its contracted state. Such sensor may be also used to determine, whether the respective lateral inflow hole is covered or closed by the struts or by the protrusions arranged at the struts as mentioned above in an easy way.

In one embodiment, the inflow cannula comprises an electrical connector used for the Peltier element and/or the sensor, which is further adapted to transmit information. The electrical connector might be a type of wiring, wherein any kind of electrical connection to the Peltier element or the sensor is inevitable for its functioning, for example due to its connection to a power supply. This electrical connector may further be used to transmit any information provided by the Peltier element or the sensor.

The received information provided by the Peltier element or the sensor may trigger an acoustic, visual and/or tactile warning signal to the health care person, for example when the cage expands or arrives in its contracted state.

In one embodiment, the sheath of the inflow cannula may comprise at least one recess on an inner surface for receiving at least one strut of the at least one cage. The recess or recesses may be fully or partially circumferential. When the sheath is in its first position, the one recess receives at least one strut. This has the advantage, that a small initial force is required to retract the sheath. And thus, the sheath may not be unintentionally displaced during the insertion or withdrawal of the inflow cannula. Instead of recesses, protrusions, grooves, bulges, small teeth or similar elements may be provided at the cage and/or tubular body and/or sheath for the aforementioned purpose.

In one embodiment the tubular body comprises a plurality of predefined sections, wherein each section comprises at least one lateral hole and at least one cage. Such inflow cannula is preferably used for venous treatment. In this configuration, each predefined section comprises one lateral hole or a plurality of lateral holes. Having multiple sections of lateral holes may increase the flow rate, while using at least one cage per section allows to use cages with a smaller maximum diameter, which results in a smaller expansion of the vessel, ventricle, vein etc.. In this embodiment, there may be one sheath covering the plurality of cages in their contracted state.

The present invention will now be described in further detail with reference to the accompanying schematic drawings, wherein
- Fig. 1: shows a side view of an inflow cannula known from the prior art,
- Fig. 2: shows a partly transparent side view of an inflow cannula according to one embodiment of the invention prepared for insertion or withdrawal, wherein a sheath is in its first position,
- Fig. 3: shows a partly transparent side view of the inflow cannula of Fig. 2, wherein its sheath is in its second, retracted position,
- Fig. 4: shows a partly transparent side view of a second embodiment of the inflow cannula, wherein the sheath comprises recesses or the like and is in its second, retracted position,
- Fig. 5: shows a partly transparent side view of a third embodiment of the inflow cannula further comprising a Peltier element and sensors, wherein the sheath is in its first position,
- Fig. 6: shows a partly transparent side view of the embodiment of Fig. 5, wherein the sheath is in its second, retracted position,

- Fig. 7: shows a partly transparent side view of a fourth embodiment of the inflow cannula, wherein the sheath is in its first position, particularly for venous medical treatment.

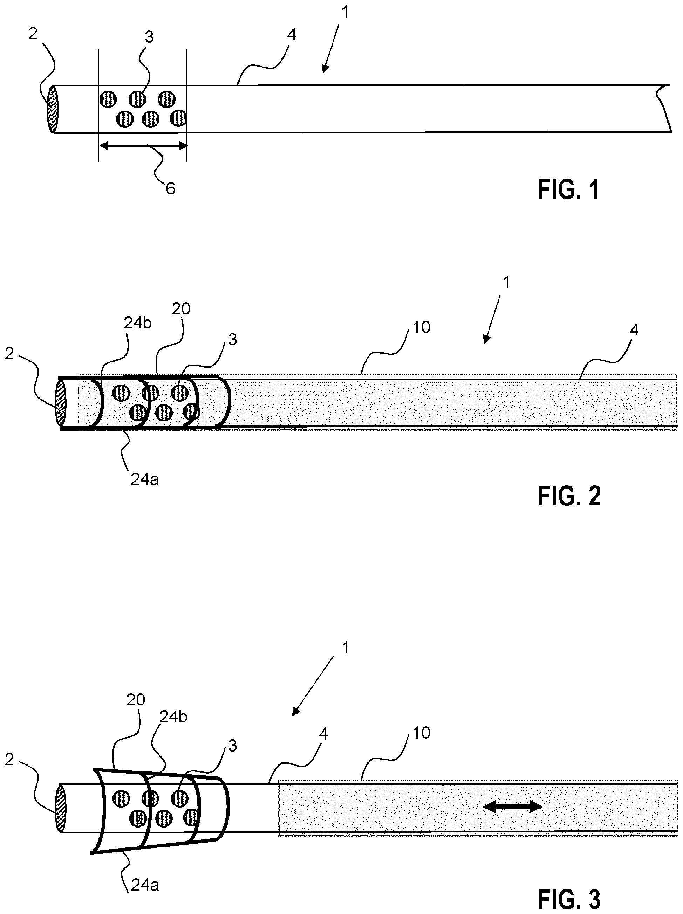

Fig. 1 shows an inflow cannula 1 known from the prior art. A tubular body 4 of the cannula comprises a distal end with a head-on hole 2 at its front end and a plurality of lateral holes 3 within a predefined section 6 of the tubular body arranged at the side of the body. The opposite distal end of the tubular body 4 is not shown. During the medical treatment, bodily fluid is extracted through the head-on hole 2 and lateral holes 3 of the cannula 1. The lateral holes 3 can be arranged circumferential as well as formed in any shape, number, or range along a tubular body 4 of the cannula 1. Further, the distal end of the cannula 1 may also comprise multiple head-on holes 2. Embodiments without any head-on hole 2 may be used as well.

Fig. 2 to 6 shows an inflow cannula 1 according to the invention, adapted, for example, for aterial application. The cannula 1 comprises the tubular body 4 and the holes 2,3 of the cannula shown in Fig. 1 as well as a cage 20 and a sheath 10. The sheath 10 has the form of a hollow cylinder and is arranged concentrically around the tubular body 4. The sheath 10 in Fig. 2 is shown in its first position, wherein the first position is defined as a position wherein the sheath 10 at least partially covers the cage 20. Therefore, the cage 20 is shown in its contracted state having generally the form of a hollow cylinder. The cage depicted in Fig. 2 comprises struts wherein a first plurality of struts 24a extend in the longitudinal direction and a second plurality of struts 24b extend in the circumferential direction of the tubular body 4. The number of struts 24 in either direction is not limited and can be adapted according to the desired medical treatment of the inflow cannula 1. The first plurality of struts 24a extending in longitudinal direction connects the second plurality of struts 24b extending in circumferential direction thereby forming the cage 20. As indicated above, different strut orientation is possible, as well.

Further, the sheath 10 is made of a biocompatible material, for example a biocompatible polymer, such as silicone.

After the sheath 10 is at least partially retracted, the cage 20 expands into a conical form. Fig. 3 depicts the cage 20 in its expanded state. Regardless of the conical shape and the number of parts of the cage 20, Fig. 3 shows that the diameter of the cage 20 at its distal end is greater than at its proximal end. The double-headed arrow shown in Fig. 3 is depicted to show the movement of the sheath 10 in longitudinal direction.

The following dimensions of the tubular body 4, the cage 20 and the sheath 10 may be useful. The tubular body may have a diameter in the range of 4 Fr (French) to 51 Fr. The cage may have a length in the range of 0.5 cm to 10 cm. The cannula may have a length in range of 10 cm to 110 cm.

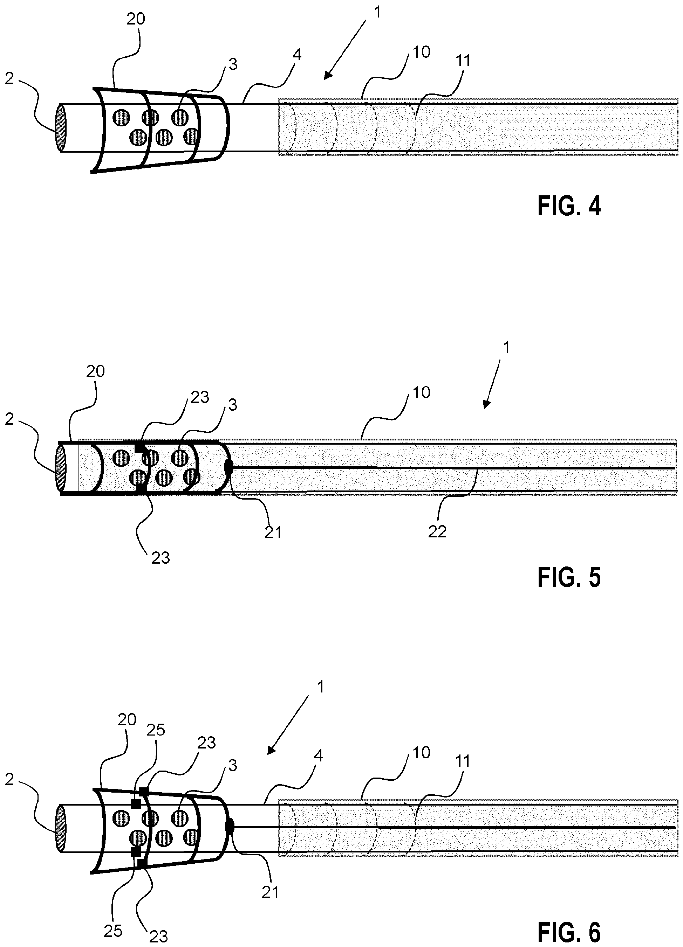

The sheath 10 may further comprise recesses 11 as shown in Fig. 4. The recesses 11 are located at the inner surface of the sheath 10 and fully extend in circumferential direction. Once the sheath 10 is in its first position, i.e. its distal position, each recess 11 receives one strut 24b of the second plurality of struts 24b of the cage 20. Therefore, an initial force is required to disengage the strut 24b from the recess 11 and therefor the sheath 10 from the cage 20. The same applies to the re-engagement of the cage 20 and the sheath 10 during re-sheathing.

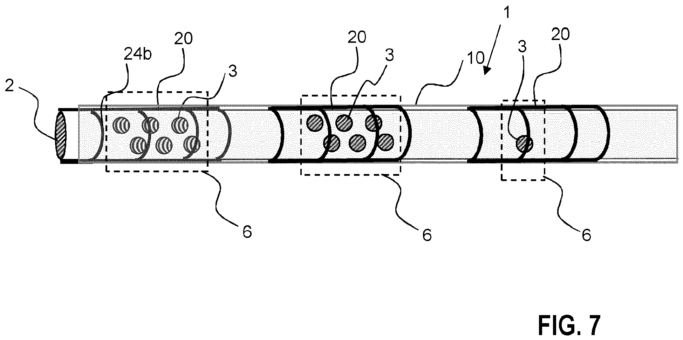

In the embodiment shown in Fig. 7 the tubular body 4 comprises a plurality of predefined sections 6 with lateral holes 3 and, accordingly, a plurality of cages. The single sheath 10 is formed integrally. The embodiment of Fig. 7 is used for medical treatment in venous vessels, for example.

Furthermore, the inflow cannula 1 may comprise a Peltier element 21 and/or sensors 23 as shown in Fig. 5. The Peltier element 21 comprises an electrical connector 22 in order to connect the Peltier element 21 to a power supply (not shown). The electrical connector is arranged between the sheath 10 and the tubular body 4. The electrical connector 22 may also be used to transmit information. Depending on the type of sensor 23, there may be a similar electrical connection to a power supply or there may be no electrical connection needed since it may be powered wirelessly.

The Peltier element 21 is attached and thermally connected to the cage 20 and thereby changes the temperature of the cage 20 when activated, i.e. the Peltier element 21 cools the cage 20 or heats it. As the cage 20 comprises a shape memory or superelastic material such as Nitinol, the change of temperature changes the flexibility and elasticity the material and thus of the cage 20. This may be of advantage, when the cage 20 is transferred from its contracted state into its expanded state or vice versa.

Once the sheath 10 is retracted along the tubular body 4 of the cannula 1, the cage 20 expands as depicted in Fig. 6. As shown in Fig. 6, the tubular body 4 comprises contact elements 25 at its outer surface formed by an electrically conducting layer or pad. Each contact element 25 is adapted to form an electrical connection with one sensor 23 if the cage 20 is in its contracted state (see Fig. 5). The electrical connection is open if the cage is in its expanded state (see Fig. 6). Thereby, the sensor 23 determines the transition of the cage 20 from its expanded into its contracted state, i.e. the transition from the configuration shown in Fig. 6 to the configuration depicted in Fig. 5.

It is obvious, that all components and features mentioned in the description can be installed individually and do not necessarily be installed in the combination as depicted in any of the Fig. 2 to 7.

The inventive inflow cannula 1 with the conical cage 20 provides space between the lateral inflow holes 3 of the tubular body 4 and the anatomical structures located near the lateral holes 3 during medical treatment. By the cage 20 and the sheath 10 the diameter of the cannula 1 is only minimally increased. The proposed conical cage 20 can be produced easily and cost-effectively due to its simple construction.

### Reference numerals

- 1: inflow cannula
- 2: head-on inflow hole
- 3: lateral inflow hole
- 4: tubular body
- 6: predefined section
- 10: sheath
- 11: recess
- 20: cage
- 21: Peltier element
- 22: electrical connector
- 23: sensor
- 24a: struts extending in longitudinal direction (first plurality)
- 24b: struts extending in circumferential direction (second plurality)
- 25: contact element

## Claims

1. An inflow cannula (1) comprising
a tubular body (4) having at least one lateral hole (3) positioned at a predefined section (6) of the tubular body (4), particularly at the distal end of the tubular body (4)
wherein the inflow cannula (1) further comprises at least one cage (20) and a sheath (10) concentrically arranged around the tubular body (4), wherein the sheath (10) is movable between a first position and a second position,
wherein the at least one cage (20) has a contracted state and an expanded state,
wherein in its expanded state the at least one cage (20) takes a conical form and is positioned in the predefined section (6) of the tubular body (4),
wherein in its contracted state the at least one cage (20) is arranged between the outer surface of the tubular body (4) and the sheath (10) when the sheath (10) is in its first position, and
wherein the cage (20) is adapted to expand and takes its expanded state when the sheath (10) is moved from its first position into its second position.

2. The inflow cannula according to claim 1, wherein the cage (20) comprises struts (24).

3. The inflow cannula according to claim 2, wherein a first plurality of struts (24a) extending in a longitudinal direction of the tubular body (4) and a second plurality of struts (24b) extending in a circumferential direction of the cage (20).

4. The inflow cannula according to any of the preceding claims, wherein the cage (20) comprises a shape memory and/or superelastic material, particularly Nitinol.

5. The inflow cannula according to any of the preceding claims, further comprising a Peltier element (21) thermally connected to the cage (20).

6. The inflow cannula according to any of the preceding claims, further comprising at least one sensor (23) and/or transmitter attached to the cage (20) or the outer surface of the tubular body (4).

7. The inflow cannula according to claim 6, wherein the tubular body comprises at least one contact element (25), wherein the at least one sensor (23) is adapted to detect an electrical connection of the at least one cage (20) with the at least one contact element (25).

8. The inflow cannula according to any of claims 5 to 7, wherein an electrical connector (22) used for the Peltier element (21) and/or the sensor (23) is adapted to transmit information.

9. The inflow cannula according to any of the preceding claims, wherein the sheath (10) comprises at least one recess (11) on an inner surface for receiving at least one strut (24a, 24b) of the at least one cage (20).

10. The inflow cannula according to any of the preceding claims, wherein the tubular body (4) comprises a plurality of predefined sections (6), wherein each section comprises at least one lateral hole (3) and at least one cage (20).
